# EUROPEAN PATENT APPLICATION

(11) **EP 4 011 393 A1**
(43) Date of publication of application: **15.06.2022**
(21) Application number: 20850280.7
(22) Date of filing: 04.08.2020
(51) Int. Cl.: A61K 45/00

(54) **PHARMACEUTICAL AGENT FOR TRANSMUCOSAL ADMINISTRATION**

(30) Priority: 05.08.2019 JP 2019143948
(71) Applicant: Denka Company Limited, Tokyo 103-8338 (JP); National University Corporation Kumamoto University, Kumamoto-shi, Kumamoto, 860-0862 (JP)
(72) Inventor: MISUMI, Shogo, Kumamoto-shi, Kumamoto 862-0973 (JP); KISHIMOTO, Naoki, Kumamoto-shi, Kumamoto 862-0973 (JP); MITSUMATA, Ryotaro, Gosen-shi, Niigata 959-1836 (JP)
(74) Representative: Blodig, Wolfgang
(86) International application number: PCT/JP2020/029836
(87) International publication number: WO 2021/025020

(57) **Abstract**

Provided is a pharmaceutical agent for enhancing absorption efficiency of a drug through the mucosal epithelial layer. A pharmaceutical agent for transmucosal administration comprises as an active ingredient a drug compound to which a podocalyxin targeting molecule is bound.

## Description

### Field of the Invention

The present invention relates to a pharmaceutical agent for transmucosal administration which excels in drug delivery performance.

### Background of the Invention

Preparations for transmucosal administration are used for treating and preventing various diseases, and preparations for oral administration particularly account for the majority of pharmaceutical products due to the convenience and the low invasiveness when taking a drug. However, in preparations for transmucosal administration, the mucosal epithelial cell layer functions as a lipid-soluble barrier, and thus a drug with a low molecular size and relatively high lipid-solubility is absorbed efficiently through the mucous membrane, but a drug with a high molecular size or an extremely water-soluble drug fails to pass through the mucosal epithelial layer and is not absorbed in the body. Accordingly, any drugs with poor absorption through the mucous membrane despite excellent pharmacological activities of these compounds cannot be used in preparations for transmucosal administration including preparations for oral administration.

Examples of the method for improving the membrane permeability of a drug with low absorption through the mucous membrane include use of an absorption enhancer and prodrug modification. The absorption enhancer can enhance the permeability of a drug through intercellular spaces by loosening tight junctions of the mucosal epithelial cell layer. However, the absorption enhancers represented by surfactants, bile acids, and fatty acids are often associated with mucosal injuries and also enhance the mucosal permeability of the substances other than a drug present on the mucous membrane surface, thereby incurring a high risk of unexpected side effects, due to which there are only a few examples of such use in clinical settings.

On the other hand, the prodrug modification improves the absorption by chemically modifying a drug itself. The prodrug modification enhances the absorption at the mucous membrane by chemically modifying a drug and is designed in such a way that a prodrug reverts to its parent drug to exert the effect after the prodrug is reached inside the body and metabolized. Examples include bacampicillin in which ampicillin is modified to enhance the lipid solubility. However, in recent years, proteinaceous drugs such as biopharmaceuticals have been commonly distributed, and these drugs are highly water-soluble high molecular compounds and thus poor in the mucosal permeability, thereby making it very difficult to apply the chemical modification for facilitating the absorption.

Vaccine preparations have long been using, as an active ingredient, an attenuated pathogen, an inactivated pathogen, or a pathogen component, and these are preparations comprising, as the chief ingredient, a protein with larger particle size and molecular size when compared with antibody pharmaceutical products and the like. Most of the vaccines to be approved are injections, but many pathogens invade through the mucous membrane, and thus if an immune response at a local mucous membrane in addition to the systemic immune response can be enhanced by a mucosal vaccine, a more effective vaccine can be expected to be created. However, the mucosal vaccines which have been already approved are infectious attenuated vaccines or vaccines through an antigen such as a toxin having the binding activity to the mucous membrane, and an inactivated antigen with no mucosal affinity is poorly taken in through the mucous membrane, thereby making it difficult to induce sufficient immune response by administration of such an antigen alone.

On the other hand, podocalyxin (PCX) which was discovered in renal glomerular epithelial cells is a glycoprotein having a molecular weight of about 140 kDa and known to be involved in the adhesion and morphogenesis of cells, and cancer progression and the like (Non Patent Literatures 1 and 2).

However, it is not known at all that podocalyxin is relevant to the mucosal absorption of drugs.

Non Patent Literature 1: Sizemore S, Cicek M, Sizemore N, Ng KP, Casey G. Podocalyxin increases the aggressive phenotype of breast and prostate cancer cells in vitro through its interaction with ezrin. Cancer Res. 2007 Jul 1; 67(13):6183-91.

Non Patent Literature 2: Takeda T, McQuistan T, Orlando RA, Farquhar MG. Loss of glomerular foot processes is associated with uncoupling of podocalyxin from the actin cytoskeleton. J Clin Invest. 2001 Jul; 108 (2) :289-301.

### Summary of the Invention

### Technical Problem

The present invention relates to a provision of a pharmaceutical agent for enhancing absorption efficiency of a drug through the mucosal epithelial layer.

### Solution to Problem

The present inventors conducted extensive studies and found that podocalyxin is expressed at a high level in microfold cells (M cell) of the mucosal epithelium. Further, the present inventors found that when a podocalyxin targeting molecule is bound to a substance such as a drug, the absorption of such a substance through the mucous membrane is surprisingly facilitated.

That is, the present invention relates to the following 1) to 8).
1) A pharmaceutical agent for transmucosal administration, comprising as an active ingredient a drug compound to which a podocalyxin targeting molecule is bound.
2) A composition for transmucosal administration, comprising a drug compound to which a podocalyxin targeting molecule is bound.
3) The pharmaceutical agent for transmucosal administration according to 1), wherein the podocalyxin targeting molecule is an anti-podocalyxin antibody or an antigen binding fragment thereof.
4) The composition for transmucosal administration according to 2), wherein the podocalyxin targeting molecule is an anti-podocalyxin antibody or an antigen binding fragment thereof.
5) The composition for transmucosal administration according to 2) or 4), wherein the composition is for transnasal inhalation, transpulmonary inhalation, tracheal or bronchial inhalation, or ocular instillation.
6) Use of a drug compound to which a podocalyxin targeting molecule is bound for manufacturing a pharmaceutical agent for transmucosal administration.
7) A drug compound for transmucosal administration, to which a podocalyxin targeting molecule is bound.
8) A method for transmucosal administration, comprising administering a drug compound to which a podocalyxin targeting molecule is bound to a mucous membrane of human or a mammal other than human.

### Advantageous Effects of the Invention

According to the present invention, it is expected that the absorption efficiency of drugs which are poorly absorbed from mucous membrane is improved to enable transmucosal administration of such drugs that could not be achieved with non-transmucosal preparations, thereby improving the enhancement of convenience and efficacy of medication.

### Brief Description of Drawings

[Figure 1A] Figure 1A shows expression confirmation of podocalyxin in a Caco-2 monolayer. A MERGE image is a composite image of a DAPI image and a GP2 image.
[Figure 1B] Figure 1B shows expression confirmation of podocalyxin in *in vitro* M-like cells. A MERGE image is a composite image of a DAPI image and a GP2 image.
[Figure 2] Figure 2 shows expression confirmation of podocalyxin in intestinal M cells in cynomolgus monkeys.
[Figure 3] Figure 3 shows the results of evaluation of the transcytosis of an anti-podocalyxin antibody-labelled fluorescent beads.

### Detailed Description of the Invention

Hereinafter, preferred embodiments of the present invention will be described in detail. However, the present invention is not limited to the following embodiments.

In the present invention, podocalyxin (PCX) refers to a type I transmembrane protein expressed in renal glomerular epithelial cells and consists of 558 amino acid residues.

The podocalyxin targeting molecule in the present invention means a molecule having an ability to bind to podocalyxin, preferably an ability to specifically bind to podocalyxin. The chemical species of the target molecule is not particularly limited and can include various chemical species such as low molecular compounds, high molecular compounds, biological substances. Specifically, examples include saccharides, lipids, oligopeptides, proteins, and nucleic acids. Examples of the function of target molecule include a podocalyxin-recognizing antibodies, lectins, and interacting proteins. Examples of the podocalyxin targeting molecule include preferably anti-podocalyxin antibodies or antigen binding fragments thereof, and lectins (for example, rBC2LCN).

The anti-podocalyxin antibody can be a monoclonal antibody or a polyclonal antibody. Further, the anti-podocalyxin antibody can be any isotype of IgG, IgM, IgA, IgD, and IgE. Also acceptable antibodies are those created by immunizing non-human animals such as mice, rats, hamsters, guinea pigs, rabbits or chickens, recombinant antibodies, chimeric antibodies, humanized antibodies, and fully humanized antibodies.

The antigen binding fragment of an anti-podocalyxin antibody means a fragment of the anti-podocalyxin antibody, the fragment binding to podocalyxin. Specifically, examples include a Fab consisting of VL, VH, CL, and CH1 regions, F(ab)2 in which 2 Fabs are linked by a disulfide bond at a hinge region, Fv consisting of VL and VH, scFv, which is a single-chain antibody in which VL and VH are linked by an artificial polypeptide linker, and additionally bispecific antibodies such as diabody type, scDb type, tandem scFv type, and leucine zipper type.

Examples of the drug compound in the present invention include drugs categorized into antipyretics, pain killers, anti-inflammatory drugs, anti-rheumatoid drugs, soporific drugs, sedative drugs, anti-anxiety drugs, antipsychotic drugs, antidepressants, antiepileptic drugs, antiparkinsonian drugs, cerebral circulation metabolism ameliorators, muscle relaxants, autonomic nervous system agonists, anti-vertiginous drugs, migraine drugs, cardiotonic drugs, antianginal drugs, β blockers, Ca antagonists, antiarrhythmic drugs, diuretics, antihypertensive drugs, antiallergic drugs, bronchodilators, antiasthmatic drugs, antitussive drugs, expectorants, peptic ulcer drugs, antipodagrics, antihyperlipidemic drugs, antidiabetic drugs, hormone preparations, osteoporosis drugs, antibacterial drugs, antiviral drugs, antiparasitics, antiprotozoal drugs, immunosuppressants, anesthesia and vaccines, and these are preferably applied to peptide or proteinaceous pharmaceutical products and water-soluble high molecular pharmaceutical products which are poorly absorbee when orally administered.

In the present invention, examples of the binding of a drug compound to a substance targeting podocalyxin include bindings via an amino group, a sulfhydryl group, a carboxyl group, and the aldehyde group generated at a reducing sugar terminus and in the sugar chain by periodate oxidation. Specifically, the binding can be achieved via crosslinking agents having an N-hydroxysuccinimide ester and an isothiocyanate group for the amino group, crosslinking agents having a maleimide group and a bromoacetamide group for the sulfhydryl group, and crosslinking agent having a hydrazide group for the aldehyde. Further, for the carboxy group, the binding can be achieved by using an N-hydroxysuccinimide ester to convert the carboxyl group to an active ester, to which a crosslinking agent having a primary amine is reacted, but the method is not limited to these.

The transmucosal administration in the pharmaceutical agent for transmucosal administration of the present invention refers to a dosage form by which the pharmaceutical agent is absorbed via the mucous membrane of the body cavity. Specifically, examples include transnasal inhalation via the mucous membrane lining the nasal cavity, ocular instillation onto the conjunctiva or the cornea, transpulmonary inhalation via the mucous membrane of the alveolus, inhalation to the tracheal mucosa or bronchial mucosa, vaginal administration via the mucous membrane in the vagina, oral administration and rectal administration via the mucous membrane of the digestive tracts such as the stomach, duodenum, small intestines, large intestines including colon and rectum, and middle ear administration to the middle ear mucous membrane. Of these, preferred dosage forms are transnasal inhalation, transpulmonary inhalation, tracheal or bronchial inhalation, and ocular instillation.

As shown in examples later, when an anti-podocalyxin monoclonal antibody is bound to fluorescent beads, the uptake by the transcellular transport in *in vitro* M-like cells, which is an intestinal M cell model, is enhanced when compared with fluorescent beads to which an isotype control is bound. That is, when a podocalyxin targeting molecule is bound to a drug compound, the transmucosal absorption of such a drug compound is enhanced, and thereby higher drug efficacy is expected compared with the administration of the drug compound alone.

Thus, the drug compound to which a podocalyxin targeting molecule is bound can be a pharmaceutical agent for transmucosal administration and used for the transmucosal administration.

The pharmaceutical agent for transmucosal administration of the present invention can be formulated into a composition (pharmaceutical composition) together with a pharmaceutically acceptable carrier for the mucosal administration in the form of solid, semi-solid or liquid.

The dosage form of such a composition is not particularly limited as long as it can be dissolved or disintegrated on the mucous membranes of various body cavities and can be a form according to a site to be the administration site. That is, the composition can be liquids (including suspensions and amorphous gels) fillable in containers such as tubes and capsules in addition to films, tablets, and dry powders.

For example, when the delivery to the mucous membrane of the respiratory tract (for example, nose) is intended, the composition is typically formulated into an aqueous solution for the administration as an aerosol or a nasal spray, or formulated into, for example, a dry powder to be quickly deposited in the nasal passage.

Examples of the liquid include those in which the composition is dissolved in purified water or a buffer solution. Examples of the suspension include those in which the composition is suspended in purified water or a buffer solution together with methyl cellulose, hydroxy methyl cellulose, polyvinylpyrrolidone, gelatin, casein or the like.

Examples of the film-like preparation and tablet-like preparation include formed products consisting of water-soluble matrix materials (for example, pullulan, polyvinyl alcohol (PVA), hydroxypropyl cellulose (HPC), polyvinylpyrrolidone (PVP), gelatin, and starch) and having a laminate structure comprising a mucoadhesion layer.

Such a preparation composition can comprise one or more typically contained excipients such as preservatives, viscosity modifiers, tonicity agents, buffering agents, absorption enhancers, surfactants, stabilizers, desiccating agents, and solubilizing agents.

To the dry powder preparation, fillers and acting substances for providing suitable features in the powder flow and size (for example, mannitol, sucrose, trehalose, and xylitol) can be suitably blended in addition to mucoadhesive substances.

The amount of the active ingredient (a drug compound to which a podocalyxin targeting molecule is bound) which can be contained in the pharmaceutical agent for mucosal administration of the present invention is not particularly limited as long as it is an amount sufficient to demonstrate the efficacy, and the amount can be suitably determined in consideration of the binding ratio to a podocalyxin targeting molecule to be used in combination.

Examples of the administration subject of the pharmaceutical agent for transmucosal administration of the present invention include human and mammals excluding human, with human being preferable. Examples of the mammals excluding human include mice, rats, hamsters, guinea pigs, rabbits, pigs, cows, goats, sheep, dogs, cats, rhesus monkeys, cynomolgus monkeys, orangutans, and chimpanzees.

### Example

Hereinafter, the present invention will be specifically described but is not at all limited thereto. Reference Example 1 Expression confirmation of podocalyxin in *in vitro* M-like cells

As described in a literature (Kai H, Motomura Y, Saito S, Hashimoto K, Tatefuji T, Takamune N, Misumi S. Royal jelly enhances antigen-specific mucosal IgA response. Food Sci Nutr. 2013 Mar 6; 1(3):222-227.), 3 x 10⁵ Caco-2 cells were seeded on the membrane of Transwell (Corning, pore size: 3 µm, 24 wells), allowed to stand overnight, and then the Transwell membrane was immersed in the 24-well plate to which Eagle's MEM comprising 20% fetal bovine serum and 0.1 mM non-essential amino acid (20% FBS, 0.1 mM NEAA EMEM) was added. By transferring the Transwell about every 3 days to the 24-well plate to which fresh 20% FBS, 0.1 mM NEAA EMEM was added, the cells on the membrane were cultured for 21 days to form a monolayer of Caco-2 cells. After 21 days of culture, 1 x 10⁶ Raji B cells were added to the upper chamber of Transwell, and the culture was continued 3 more days to induce the differentiation to M-like cells. During this operation, Transwell which was not co-cultured with Raji B cells was also created as a control (control: Caco-2 monolayer).

After 3 days of co-culture with Raji B cells, the Transwell membrane was cut off and immobilized by being immersed in methanol for 10 minutes, and then masking was carried out with 5% skim milk-containing D-PBS. An anti-podocalyxin antibody (R&D Systems) was 40-fold diluted with skim milk-containing D-PBS, the membrane after masking was immersed therein, and subsequently immersed in skim milk-containing D-PBS which contains an Alexa488-labeled donkey anti-goat IgG antibody and an Alexa555-labeled anti-GP2 antibody, and diamino-phenyliodide (DAPI). The stained membrane was observed using a laser microscope (Keyence).

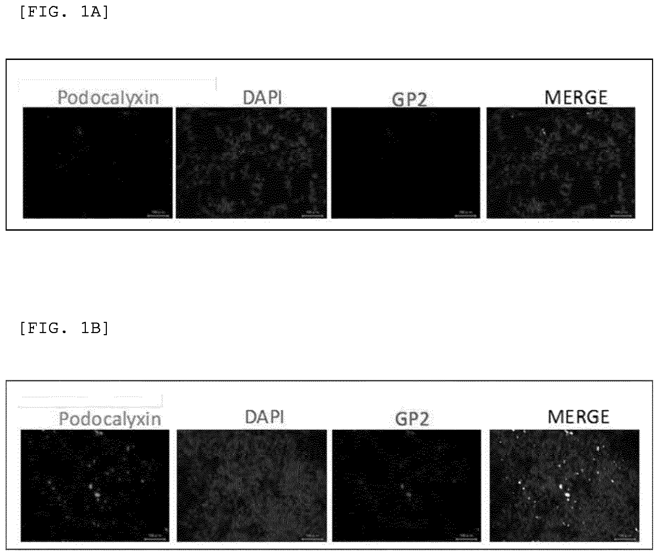

Figure 1A shows microscopic images of the membranes which were not co-cultured with Raji B cells, and as the differentiation to M cells was not induced, neither podocalyxin nor GP2, an M cell marker, was substantially confirmed. To the contrary, when co-cultured with Raji B cells and induced to differentiate to M cells, not only the GP2 expression was enhanced but also the podocalyxin expression was enhanced as shown in Figure 1B, thereby it was confirmed that podocalyxin was colocalized with GP2 which is the M cell marker. Thus, it was considered that podocalyxin is expressed in the M cells which were differentiated *in vitro.*

### Reference Example 2 Expression confirmation of podocalyxin in cynomolgus monkey intestinal M cells

The portion from 30 cm toward the ileum from the cecum up to the cecum of a cynomolgus monkey was excised and embedded in OCT Compound (Sakura Finetek Japan Co., Ltd.) to produce a frozen section. The frozen section comprising a Peyer's patch was immersed in cold acetone to carry out fixation treatment and then immersed for 3 hours in 5% skim milk-containing D-PBS to carry out masking treatment. After the masking, staining was carried out using skim milk-containing D-PBS comprising an anti-podocalyxin antibody (R&D Systems), an Alexa488-labeled donkey anti-goat IgG antibody, an Alexa555-labeled anti-GP2 antibody, and diamino-phenyliodide (DAPI). The stained tissue sections were observed using a laser microscope (Keyence).

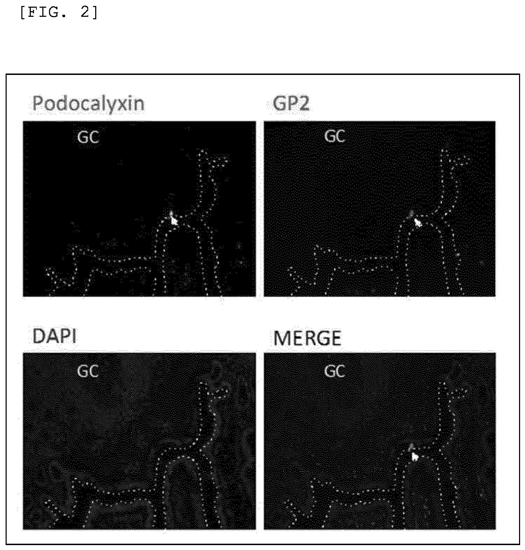

Figure 2 shows the laser microscopic images, also confirming the expression of podocalyxin at the parts shown by arrows in the figure together with the expression of GP2, which is an M cell marker. Thus, it was confirmed that podocalyxin is also expressed even in the intestinal M cells of a cynomolgus monkey.

### Example 1 Evaluation of transcytosis by anti-podocalyxin antibody-bound beads

200 pmol of biotin-labeled goat anti-mouse IgG antibody (Jackson ImmunoResearch) was collected as biotin, 950 µL of PBS was added thereto, and FluoSpheresTM Streptavidin-Labeled Microspheres equivalent to 0.1 mg was further added gradually. After the addition, 1-hr shaking was carried out while blocking out light to prepare fluorescent beads to which the anti-mouse IgG antibody was bound.

To the fluorescent beads to which the anti-mouse IgG antibody was bound, 200 µL of PBS was added, and 176 pmol of an anti-podocalyxin antibody (manufactured by Denka Seiken Co., Ltd., clone name: 4D2) or a mouse IgG2a isotype control (Sigma Aldrich) was further added, and the resultant was shaken for 1 hour while shielded from light. After the shaking, centrifugation was carried out at 15,000 rpm for 30 minutes and the beads were suspended by adding, to the precipitation, MEM (GIBCO) in which 1.3 mL of 20% fetal bovine serum, 1% NEAA, and 0.0293% L-glutamine were added, to prepare anti-podocalyxin antibody-labeled fluorescent beads or isotype control-labeled fluorescent beads.

The cells induced to differentiate to M-like cells *in vitro* as described in Reference Example 1 were prepared on the membrane of Transwell, the above prepared anti-podocalyxin antibody-labeled fluorescent beads or isotype control-labeled fluorescent beads were added to the lower chamber thereof, and the amounts of beads moved to the upper chamber after 1, 2 and 3 hours from the addition were evaluated by measuring fluorescence intensities of the upper chamber solution.

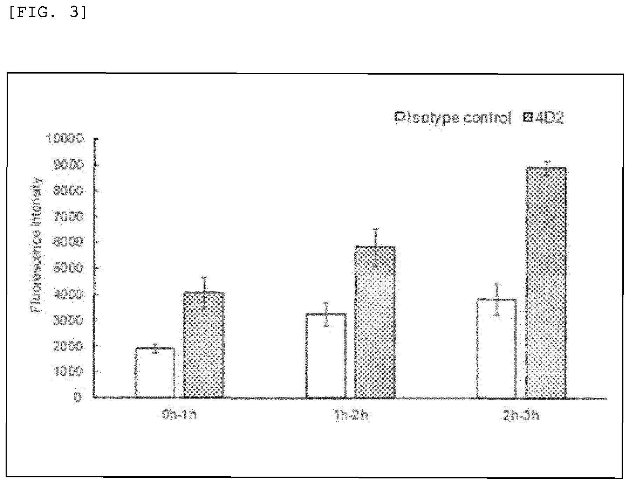

Figure 3 shows the measurement results of fluorescence intensity in the upper chamber of each fluorescent beads, and the anti-podocalyxin antibody-labeled fluorescent beads had higher fluorescence intensities in the upper chamber than the isotype control at any time of the samplings of 1, 2 and 3 hours later. This suggests that the anti-podocalyxin antibody-labeled fluorescent beads had better transport efficiency by the transcytosis via M cells from the lower chamber to the upper chamber. Thus, it is considered that a substance to which a podocalyxin targeting molecule is bound has enhanced absorption efficiency through the mucous membrane via the transcytosis of M cells.

## Claims

1. A pharmaceutical agent for transmucosal administration, comprising as an active ingredient a drug compound to which a podocalyxin targeting molecule is bound.

2. A composition for transmucosal administration, comprising a drug compound to which a podocalyxin targeting molecule is bound.

3. The pharmaceutical agent for transmucosal administration according to claim 1, wherein the podocalyxin targeting molecule is an anti-podocalyxin antibody or an antigen binding fragment thereof.

4. The composition for transmucosal administration according to claim 2, wherein the podocalyxin targeting molecule is an anti-podocalyxin antibody or an antigen binding fragment thereof.

5. The composition for transmucosal administration according to claim 2 or 4, wherein the composition is for transnasal inhalation, transpulmonary inhalation, tracheal or bronchial inhalation, or ocular instillation.

6. Use of a drug compound to which a podocalyxin targeting molecule is bound for manufacturing a pharmaceutical agent for transmucosal administration.

7. A drug compound for transmucosal administration to which a podocalyxin targeting molecule is bound.

8. A method for transmucosal administration, comprising administering a drug compound to which a podocalyxin targeting molecule is bound to a mucous membrane of human or a mammal other than human.
